# EUROPEAN PATENT APPLICATION

(11) **EP 3 327 704 A1**
(43) Date of publication of application: **30.05.2018**
(21) Application number: 16200658.9
(22) Date of filing: 25.11.2016
(51) Int. Cl.: G09F 3/00, B65D 25/36

(54) **A DENTAL PACKAGE AND A METHOD OF MAKING A DENTAL PACKAGE**

(71) Applicant: 3M Innovative Properties Company, Saint Paul, MN 55133-3427 (US)
(72) Inventor: Preininger, Martin, 82211 Herrsching (DE); Soeldner, Manfred, 82387 Antdorf (DE); Peuker, Marc, 86938 Schondorf (DE); Broyles, Bruce Robert, Saint Paul, Minnesota 55133-3427 (US)
(74) Representative: Hohmann, Arno

(57) **Abstract**

A method of processing a dental package (1) which has the steps of providing a dental material, providing a cartridge (10) for storing the dental material, providing a first label (20), providing material specific information that is associated with the dental material, providing a second label (30), the second label being circumferentially smaller than the first label, marking at least one of the first and second label with the material specific information; and attaching the second label onto the first label such that the material specific information is enclosed between the first and second label, and such that the second label does nowhere project over the first label.

## Description

### Field of the Invention

The invention relates to a method of processing a dental package and a dental package. In particular the invention relates to enclosing material specific information between a first and a second label on the dental package. The second label is circumferentially smaller than the first label. Further, the invention relates to a stripe which has a plurality of labels that are configured corresponding to the configuration of the first and second label.

### Background Art

Dental materials are typically provided in packages that are labelled with general information and material specific information about the dental material stored therein. Typically the general information relates to the general type of material, for example a filling material that belongs to a particular category (for example, based on a brand, an underlying general chemical composition, a general applicability for a certain type of treatment etc.), a color code, a viscosity grade or the like. The material specific information typically relates to the batch or amount of material actually stored within the dental package, for example a minimum durability of the dental material, a date of filling, a batch or lot number, an identifier of the place of manufacturing or an actual weight of an amount of material stored within the dental package.

Accordingly, the material specific information about the actually stored material may vary from package to package or from batch to batch although the same general information would be appropriate for all those packages or batches. For example, a dental package may be labelled with general information that comprises the weight unit, for example 5 g, while the actual weight of the material stored in the cartridge is 5.2 g. The actual weight may then be provided on the label as material specific information.

There is a general requirement to provide dental packages with general and material specific information in a durable manner to avoid loss information on the package and to avoid misuse resulting therefrom.

In the manufacturing of dental packages the material specific information often has to be provided on labels during the manufacturing process, in particular after the material has been filled in the dental package (or in close timely relationship thereto). As a result it is typically impossible to pre-manufacture labels at high volumes that comprise material specific information so that the material specific information has to be marked on the labels generally real time with the manufacturing of the dental packages. On the other hand printing processes that allow the manufacturing of durable labels are often based on high large scale printing while the real-time marking of information on labels piece by piece during the manufacturing often is less durable as compared to the high volume printing. This is due to the different printing technologies that can typically be used on large scale as opposed to piece by piece.

Therefore it is an object of the present invention to provide a label for a dental package which contains material specific information in a durable form.

### Summary of the Invention

The invention relates to a method of processing a dental package. The method comprises the steps of:
- providing a dental material;
- providing a cartridge for storing the dental material;
- providing a first label having a two-dimensional first format defined by a circumferential outermost first edge of the first label;
- providing material specific information that is associated with the dental material;
- providing a second label having a two-dimensional second format defined by a circumferential outermost second edge of the second label,
- wherein the first format and the second format are sized and shaped so that the second label can be entirely arranged on the first label so that no portion of the second label overlaps the first edge;
- marking at least one of the first and second label with the material specific information; and
- attaching the second label onto the first label such that the material specific information is enclosed between the first and second label, and such that the first edge and the second edge do not overlap.

The invention provides a dental package on which general information as well as material specific information about the dental material is provided at a maximized durability. In particular the dental package of invention (for example as obtained by the method of the invention) enables cleaning and/or disinfection of the dental package by use of disinfectants and/or cleaning agents, in particular liquid disinfectants and/or liquid cleaning agents. The invention provides for a protection of the general information and the material specific information by enclosing such information between the first and the second label. Accordingly even the use of such disinfectants or cleaning agents is enabled which would normally cause that information to affect or remove. Further, although two labels (the first and the second label) are attached onto one another the invention prevents the creating of undesired voids between the labels or between one of the labels and the cartridge. Such voids have has been found undesired by the inventors of the present invention because such voids cause the risk of capturing undesired substances and/or bacteria. Undesired substances may be dirt or dust or even residuals of disinfectants or cleaning agents. Due to the "undersize" of the second label relative to the first label the first and second label can be attached to each other without the creating of such voids although tolerances in positioning the first and second label relative to each other occur. In this regard, the term "undersize" refers to the fact that the first and second label are shaped and sized so that they can be attached to each other such that a portion of the first label circumferentially projects over the second label. Further, the invention is advantageous in that is facilitates the processing of dental packages. In particular, the invention allows the marking of the dental package during the manufacturing process (for example during the manufacturing stage at which the dental package is filled with the dental material), but nevertheless enables the marked information to be protected and enables the hygiene level to be maximized.

In a preferred embodiment the second label is transparent or translucent. Thus, the second label permits visible information, such as general and material specific information, provided on the first label to be seen through the first label.

In an embodiment the second label extends circumferentially beyond the material specific information. Accordingly, the material specific information and a circumferential portion around the material specific information may be entirely encapsulated between the first and the second label.

The first label may further exhibit general information, such as a branding, a type of the dental material, information about the manufacturer, a color code, a safety sign or an image. Furthermore, the material specific information may be provided on the first label within a pre-determined print area. This print area may further comprise the general information. For example the first label may be pre-printed with general information and the material specific information may be added, for example by marking, on the first label. Therefore, the second label may extend circumferentially beyond the print area that contains the general information and the material specific information.

In an embodiment the material specific information comprises one or more of a serial number, a lot number, date information, time information, shelf-life information, and/or a bar code.

In a further embodiment the method further comprises the steps of bonding the first label on the cartridge and bonding the second label on the first label. The first and second label preferably each have an adhesive side that is provided with an adhesive, preferably a contact or pressure sensitive adhesive. The first label may be bonded with the adhesive side on the cartridge and the second label may be bonded with the adhesive side on the first label. Typically the contact or pressure sensitive adhesive is transparent, at least when bonded to a surface. Accordingly, the first label including the adhesive is preferably transparent.

In an embodiment the method further comprises the step of positioning the second label relative to a landing zone indicated on the first label for bonding the second label on the first label. The landing zone may be indicated by one or more positioning marks provided (for example pre-printed) on the first label. A positioning mark may comprise a frame or corners of a frame that is sized in accordance with the shape and size of the second label. The positioning mark(s) enable(s) positioning both, by machine and manually by an operator.

In an embodiment the first and second label have a rectangular shape. The first label may have a two-dimensional first format that is characterized by a first width and a first length, and the second label may have a two-dimensional second format that is characterized by a second width and a second length. According to the invention the first format and the second format are sized and shaped so that the second label can be entirely arranged on the first label so that no portion of the second label overlaps the first edge. With respect to this embodiment this means that the second width is smaller than the first width and the second length is smaller than the first length. Thus, the first and second rectangular label can be arranged on one another so that the dimension of the first width is parallel to the dimension of the second width (or so that the dimension of the first length is parallel to the dimension of the second length). The corners of each of the first and second label may be rounded or chamfered.

In an embodiment the method further comprises the step of providing a stripe that holds a plurality of type-one labels corresponding in configuration to the first label and a plurality of type-two labels corresponding in configuration to the second label. The type-one labels and the type-two labels are preferably arranged alternately in sequence. Alternatively, the type-one labels and the type-two labels may be arranged in groups that are arranged alternately in sequence. Such arrangements enable the use of the stripe in a standard labeling equipment although labels of different configuration are processed.

In a further embodiment the method steps of the invention may form a first cycle in processing a first dental package. The method may further comprising the step of repeating the method steps of the invention as a second cycle in processing a second dental package. Further, the material specific information may vary from the first to the second package. In particular the invention may provide a method of processing a dental package, comprising the steps of:
(a) providing a dental material;
(b) providing a cartridge for storing at least a portion of the dental material;
(c) providing a type-one label having a two-dimensional first format defined by a circumferential outermost first edge of the type-one label;
(d) providing material specific information that is associated with the dental material or the portion of the dental material;
(e) providing a type-two label having a two-dimensional second format defined by a circumferential outermost second edge of the type-two label,
(f) wherein the first format and the second format are sized and shaped so that the type-two label can be entirely arranged on the type-one label so that no portion of the type-two label overlaps the first edge;
(g) marking at least one of the type-one and type-two label with the material specific information; and
(h) attaching the type-two label onto the type-one label such that the material specific information is enclosed between the type-one and type-two label, and such that the first edge and the second edge do not overlap;
(i) repeat steps (a) to (h) or (b) to (h).

The invention further relates to a stripe of labels comprising a release liner, a plurality of type-one labels each type-one label having a two-dimensional first format defined by a circumferential outermost first edge of the respective type-one label, and a plurality of type-two labels each type-two label having a two-dimensional second format defined by a circumferential outermost second edge of the respective type-two label. The first format and the second format are sized and shaped so that the type-two label can be entirely arranged on the type-one label so that no portion of the type-two label overlaps the first edge. Further, the type-one labels and the type-two labels are arranged alternately in sequence or in groups that are arranged alternately in sequence. The stripe of labels may be provided in the form of a reel, for example.

In an embodiment only one of the type-one and the type-two labels are printed with one or both of material specific information and general information. For example, only the type-one label may be printed with material specific information and general information.

The invention further relates to a dental package comprising a cartridge for storing a dental material. The dental package comprises a first label having a two-dimensional first format defined by a circumferential outermost first edge of the first label and a second label having a two-dimensional second format defined by a circumferential outermost second edge of the second label. The first format and the second format are sized and shaped so that the second label can be entirely arranged on the first label so that no portion of the second label overlaps the first edge. At least one of the first and second label is marked with material specific information that is associated with the dental material. The second label is arranged on the first label such that the material specific information is enclosed between the first and second label, and such that the first edge and the second edge do not overlap.

In one embodiment the cartridge comprises the dental material. The dental material may be selected from among a dental restorative material, a dental temporary restorative material, a dental filling material, a dental bonding material and a dental impression material. Other dental materials are possible as appropriate.

In an embodiment the dental package has a first portion of the dental material stored in a first chamber formed by the cartridge. The dental package may have a first piston that closes the first chamber at a rear end of the first chamber. The cartridge at a front end (opposite of the rear end) may further have a first outlet for the first portion of the dental material stored in the first chamber.

In a further embodiment the dental package has a second portion of the dental material stored in a second chamber formed by the cartridge. The dental package may have a second piston that closes the second chamber at a rear end of the second chamber. The cartridge at a front end (opposite of the rear end) further may have a second outlet for the second portion of the dental material stored in the second chamber. The first and the second chamber are preferably arranged parallel (side by side) to each other.

In a further embodiment the dental package further comprises at least one mixer. The mixer preferably has a first inlet and a second inlet for attaching to the first and the second outlet, respectively, and a dispensing opening for dispensing a mixture from the first and the second portion of the dental material mixed within the mixer. The mixer preferably has a mixing element, for example a static mixing element or a mixing rotor that can be motor driven from outside the mixer.

### Brief Description of the Figures

- Fig. 1: is a perspective view of a dental package according to an embodiment of the invention;
- Fig. 2: is a top view on a first and a second label as part of a dental package according to an embodiment of the invention;
- Fig. 3: is a cross-sectional view of a dental package according to an embodiment of the invention;
- Fig 4.: is a schematic view illustrating a situation that may occur in the prior art;
- Fig 5.: is a schematic view illustrating another situation that may occur in the prior art; and
- Fig. 6: is a top view of a portion of a stripe of labels as it may be used with the present invention.

### Detailed Description of the Invention

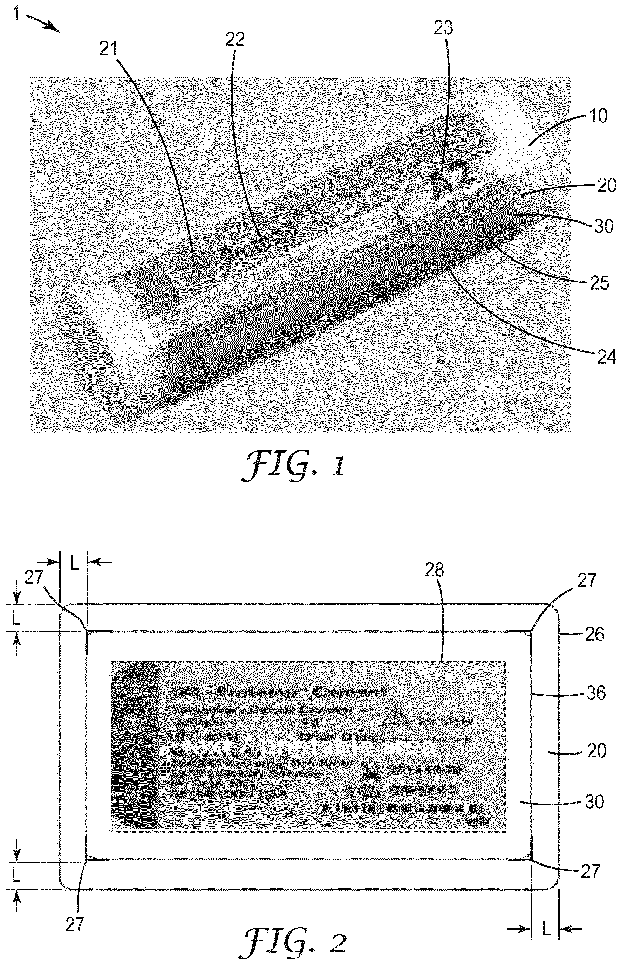

Fig. 1 shows a dental package 1 which has a cartridge 10, a first label 20 and a second label 30. The first label 20 is arranged between the cartridge 10 and the second label 30. The cartridge 10 contains a dental material (not shown). In the example, the first label 20 has general information, in particular a company name 21, a brand 22 and a color code 23 of the material. It is noted that a color code, as provided in the example, covers a particular color including tolerances so that the actual color of the dental material actually stored in the cartridge 10 may very slightly vary from the actual color of a dental material stored in a different cartridge provided under the same brand and color code. Further, the first label 20 has material specific information which relates to the material actually stored within the cartridge 10. In particular, the first label 20 has a lot number 24 which identifies from which manufacturing lot the material origins. The first label 20 is covered by a transparent second label 30. Therefore the general information as well as the material specific information are protected between the first and the second label 20, 30. It is noted that the general information and/or the material specific information may in another example be provided on the second label 30, for example on a side of the label that faces the first label 20.

The first and second label 20, 30 have different two-dimensional formats as also illustrated in more detail in Fig. 2. In particular, the first label 20 has a two-dimensional first format that is defined by a circumferential outermost first edge 26, and the second label 30 has a two-dimensional second format that is defined by a circumferential outermost second edge 36. The second format is smaller than the first format in each of the two dimensions. This means, that the second label can be arranged fully inside the boundaries defined by the first label so that the first edge and the second edge at no point overlap. In the example the first and second format are rectangular with rounded corners, although other shapes like circular, elliptical or any other shape are possible.

Further, the second label 30 is positioned on the first label 20 so that the first label 20 is uncovered at a fully circumferential area between the first and the second edge 26, 36. The first label 20 comprises positioning marks 27. The positioning marks 27 allow for positioning the second label 30 at the place marked by the positioning marks 27 on the first label 10. In the example the positioning marks are in the form of corners of a rectangle that essentially corresponds in the format to the second format of the second label 30. However, other marks like crosshairs, dots or the like may be used. The first label 20 has a print area 28. The print area 28 is defined by a two-dimension third format. The third format and the second format are sized and shaped so that the print area 28 can be entirely covered by the second label so that no portion of the print area overlaps the second edge. Therefore, the second label covers a fully circumferential area around the print area 28. This helps preventing undesired substances, for example cleaning agents or disinfection agents, from getting into contact with the printing area 28. Accordingly, any general information or material specific information arranged within the print area 28 is protected from getting into contact with such undesired substances. Thus, the general information and material specific information is maximized in durability. As described, the second label 30 is undersized relative to the first label 20 and attached to the first label 10 such that a circumferential area on the first label 20 remains uncovered. Accordingly, the first label 20 overlaps with the second label 30 and further circumferentially projects over the second edge 36. The projection has a dimension that is determined to account for tolerances of the first and the second format as well as for tolerances in the positioning of the second label 30 relative to the first label 20. The projection is greater than zero. In the example, the desired projection is 2.5 mm which enables automatic and manual positioning so that the actual projection (eventually including inaccuracies in positioning) is still greater than zero.

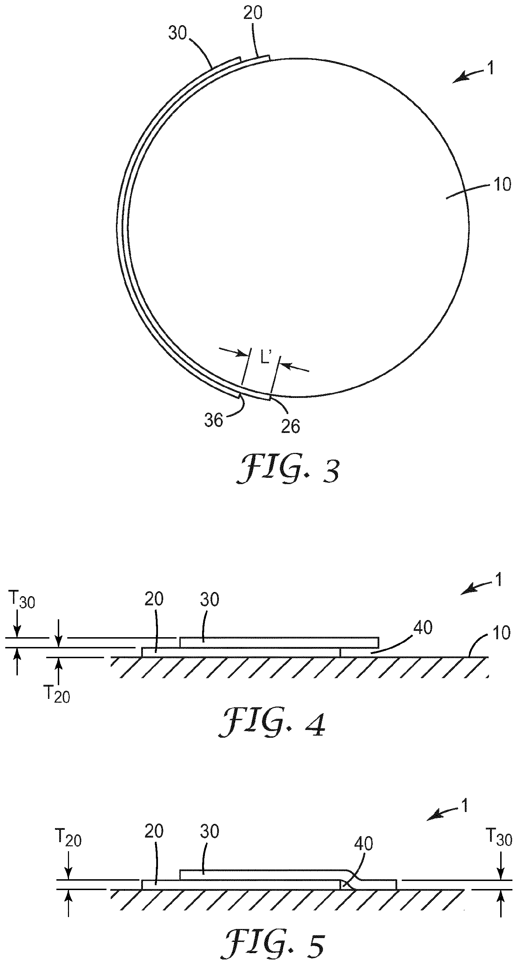

Fig. 3 shows a cross-section of the dental package 1 with the cartridge 10, and the first and the second label 20, 30 applied on the cartridge 10. As shown the projection has a dimension L' which is greater than zero. Therefore, it is prevented that the second label 30 projects over the first edge 26 and thereby it is prevented that a void is created between the second label 30 and the cartridge 10, as described in Fig. 4.

Fig. 4 shows schematically the dental package 1 with the cartridge 10, and the first and the second label 20, 30 applied on the cartridge 10. The second label 30 is incorrectly positioned relative to the first label 20 so that it projects over the first label 20. Because the first label 20 has a definite thickness T20 a void 40 is formed between the second label 20 and the cartridge 10. Typically the thickness T20 is between about 0.02 mm and 0.2 mm. Hence, the thickness is relatively small so that the void 40 formed between the second label 20 and the cartridge 10 may automatically fill with an undesired substance in case the dental package 1 is exposed to an undesired substance (for example by capillary effect in case the substance is liquid). This is avoided by the present invention.

Fig. 5 shows the same situation as Fig. 4 except than the projection of the second label 30 is attached to the cartridge 10. As illustrated due to the definite thickness T20 of the first label 20 there is still a void 40 which eventually can trap any undesired substance or bacteria. In particular, due to the fact that the second label has a definite thickness of between about 0.02 mm and 0.2 mm too, there is a reset force in the second label 30 due to the projection being bent toward the cartridge 10. Such reset force may cause the projection to detach from the cartridge 10 over time, undesired substances and/or bacteria may get into the void 40 and inadvertent attaching of the projection may cause the undesired substances and/or bacteria to be trapped in the void 40. This is avoided by the present invention too.

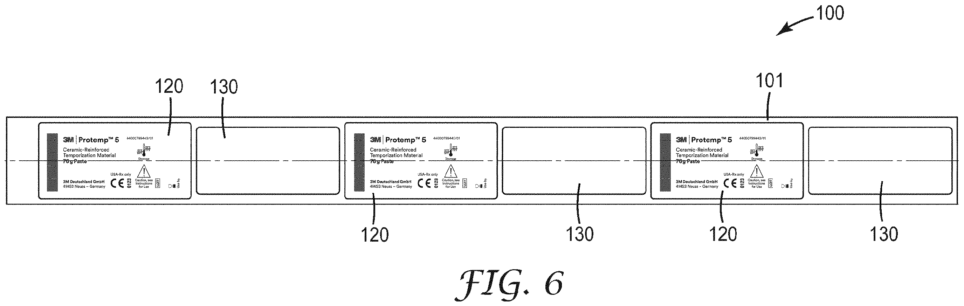

Fig. 6 shows a stripe of labels 100. The stripe of labels 100 comprises a release liner 101. The release liner 101 in the example is based on a paper or plastic band which is coated with release agent, for example a cross-linkable silicone. The release liner 101 carries a plurality of type-one labels 120 and a plurality of type-two labels 130. The type-one labels 120 correspond to the configuration of the first label described in Figures 1 to 5 and the type-two labels 130 correspond to the configuration of the second label described in Figures 1 to 5. The type-one labels 120 and the type-two labels 130 are arranged alternately in sequence.

Accordingly, in a manufacturing method of processing a dental package, the stripe of labels may be provided in a labelling station of a manufacturing machine. A type-one label 120 may be picked from the release liner 101 and placed onto a cartridge (not shown in this view). Material specific information may be marked on the type-one label 120 placed on the cartridge or at the stage at which the label is still on the stripe. A type-two label 130 may be picked from the release liner 101 and placed onto the type-one label 120 already placed on the cartridge. The steps of picking and placing a type-one label 120, marking and picking and placing a type-two label 130 may be repeated as desired. The configuration of the stripe 100 with two types of labels arranged thereon allows the use of existing labeling equipment although different labels are used.

## Claims

1. A method of processing a dental package, comprising the steps of:
- providing a dental material;
- providing a cartridge for storing the dental material;
- providing a first label having a two-dimensional first format defined by a circumferential outermost first edge of the first label;
- providing material specific information that is associated with the dental material;
- providing a second label having a two-dimensional second format defined by a circumferential outermost second edge of the second label,
- wherein the first format and the second format are sized and shaped so that the second label can be entirely arranged on the first label so that no portion of the second label overlaps the first edge;
- marking at least one of the first and second label with the material specific information; and
- attaching the second label onto the first label such that the material specific information is enclosed between the first and second label, and such that the first edge and the second edge do not overlap.

2. The method of claim 1, wherein the second label is transparent or translucent.

3. The method of claim 1 or 2, wherein the second label extends circumferentially beyond the material specific information.

4. The method of any of the preceding claims, wherein the first label exhibits general information, such as a branding, a type of the dental material, information about the manufacturer, a color code, a safety sign or an image.

5. The method of any of the preceding claims, wherein the material specific information comprises one or more of a serial number, a lot number, date information, time information, shelf-life information, and/or a bar code.

6. The method of any of the preceding claims, further comprising the steps of bonding the first label on the cartridge and bonding the second label on the first label.

7. The method of claim 6, further comprising the step of positioning the second label relative to a landing zone indicated on the first label for bonding the second label on the first label.

8. The method of any of the preceding claims, further comprising the step of providing a stripe that holds a plurality of type-one labels corresponding in configuration to the first label and a plurality of type-two labels corresponding in configuration to the second label, wherein the type-one labels and the type-two labels are arranged alternately in sequence or in groups that are arranged alternately in sequence.

9. The method of any of the preceding claims, wherein the method steps as defined in claim 1 form a first cycle in processing a first dental package, and wherein the method further comprising the step of repeating the method steps as defined in claim 1 as a second cycle in processing a second dental package, wherein the material specific information varies from the first to the second package.

10. A stripe of labels comprising a release liner, a plurality of type-one labels each type-one label having a two-dimensional first format defined by a circumferential outermost first edge of the respective type-one label, and a plurality of type-two labels each type-two label having a two-dimensional second format defined by a circumferential outermost second edge of the respective type-two label, wherein the first format and the second format are sized and shaped so that the type-two label can be entirely arranged on the type-one label so that no portion of the type-two label overlaps the first edge, and wherein the type-one labels and the type-two labels are arranged alternately in sequence or in groups that are arranged alternately in sequence.

11. The stripe of labels of claim 10, wherein only one of the type-one and the type-two labels are printed with one or both of material specific information and general information.

12. A dental package comprising a cartridge for storing a dental material, a first label having a two-dimensional first format defined by a circumferential outermost first edge of the first label and a second label having a two-dimensional second format defined by a circumferential outermost second edge of the second label, wherein the first format and the second format are sized and shaped so that the second label can be entirely arranged on the first label so that no portion of the second label overlaps the first edge, wherein at least one of the first and second label is marked with material specific information that is associated with the dental material, and wherein the second label is arranged on the first label such that the material specific information is enclosed between the first and second label, and such that the first edge and the second edge do not overlap.

13. The dental package of claim 12, further having a first portion of the dental material stored in a first chamber formed by the cartridge and having a first piston that closes the first chamber at a rear end of the first chamber, the cartridge at a front end further having a first outlet for the first portion of the dental material stored in the first chamber.

14. The dental package of claim 13, having a second portion of the dental material stored in a second chamber formed by the cartridge and having a second piston that closes the second chamber at a rear end of the second chamber, the cartridge at a front end further having a second outlet for the second portion of the dental material stored in the second chamber, and wherein the first and the second chamber are arranged parallel to each other.

15. The dental package of claim 14, further comprising at least one mixer having a first inlet and a second inlet for attaching to the first and the second outlet, respectively, and a dispensing opening for dispensing a mixture from the first and the second portion of the dental material mixed within the mixer.
